# EUROPEAN PATENT APPLICATION

(11) **EP 4 180 014 A1**
(43) Date of publication of application: **17.05.2023**
(21) Application number: 21207961.0
(22) Date of filing: 12.11.2021
(51) Int. Cl.: A61F 13/15, A61F 13/496

(54) **A METHOD AND APPARATUS FOR PRODUCING WASHABLE ABSORBENT GARMENTS**

(71) Applicant: Fameccanica.Data S.p.A., 66020 San Giovanni Teatino (CH) (IT)
(72) Inventor: LUPINETTI, Serafino, I-66020 San Giovanni Teatino (Chieti) (IT)
(74) Representative: Marchitelli, Mauro

(57) **Abstract**

A method for producing washable absorbent garments (10), wherein blank washable absorbent garments (70) are folded to overlap to each other side edges (24, 26) of rear and front waist sections (14, 16), and wherein the overlapped side edges (24, 26) are welded along respective welding lines (94) and simultaneously cut flush with the respective welding lines (94).

## Description

### Field of the invention

The present invention relates to absorbent garment for the absorption and retention of liquids, and more particularly to washable and reusable absorbent garments.

More specifically, the invention relates to a method and apparatus for producing washable absorbent garments.

### Prior art

Various washable garments having a pant-like configuration and useful as incontinence garments, menstrual garments, training garments, diapers, and the like, are commercially available, as well as being disclosed in various patents.

For instance, absorbent garments are commercially available which are machine washable, and thus reusable, having an absorbent pad sewn into the crotch area.

US 3489149 discloses a menstrual panty having a pocket sewn into the crotch area, the pocket being used for receiving an absorbent disposable menstrual pad. Similarly, US 4352356 discloses a urinary incontinence panty having a pouch connected in the crotch area and adapted to receive an absorbent disposable urinary incontinence pad. Another sanitary panty garment with a pocket in the crotch area is disclosed in US 3613686.

Also, washable absorbent garments are known wherein the absorbent pad is permanently connected to the pant structure. For instance, GB2176692A discloses an absorbent sanitary garment made in the form of knitted polyester material which incorporates a non-removable pad made of brushed polyester which has the appearance of soft felt. The absorbent pad cannot be removed and is made of a material which can be frequently washed.

Prior art washable absorbent garments are manufactured by the same techniques uses for manufacturing textiles articles, typically by manually cutting and sewing fabric materials. In the prior art washable absorbent garments a washable absorbent core is typically sewn to a fabric layer forming a pant-structure and opposite waist sections of the pant-structure are sewn to each other. The typical production rate of prior art washable absorbent garments is in the range of 1 piece/1'.

Currently all washable absorbent products on the market are closed laterally by stitching, hooks or press studs. As an alternative to the above, a seamless product is proposed, made from a continuous fabric tube which does not have closures and discontinuity elements on the sides. Closure systems wherein the side edges are overlapped and connected by glued are also present on the market of traditional underwear.

The limits of the various solutions currently on the market essentially concern the impossibility of producing the washable absorbent garments on fully automatic machines. Therefore, all the aforementioned processes require more or less important manual actions on the part of the operators, which consequently implies increased production times. increased costs and a scarce repeatability of the finished products.

In addition, many of the known solutions produce an area of increased thickness on the sides due to the presence of overlapped layers and stitched folds which have undesirable aesthetic effects especially for the users who intends to wear the washable absorbent articles underneath tight clothes.

### Object and summary of the invention

The object of the present invention is to provide a method for producing washable absorbent garment which overcomes the problems of the prior art.

According to the present invention, this object is achieved by a method having the features of claims 1.

According to another aspect, the invention relates to a washable absorbent garment according to claim 12 and to an apparatus for producing washable absorbent garments having the features of claim 13.

The claims form an integral part of the disclosure provided here in relation to the invention.

### Brief description of the drawings

The present invention will now be described in detail with reference to the attached drawings, given purely by way of non-limiting example, wherein:
- Figure 1 is a perspective view of a washable absorbent garment in an open configuration,
- Figure 2 is a plan view of the absorbent garment of figure 1 in the open configuration,
- Figure 3 is a schematic cross-section taken along the line III-III of figure 2,
- Figures 4 and 5 are schematic side views of an apparatus for producing the washable absorbent garments of figures 1-3,
- Figures 6-11 are schematic plan views showing intermediate steps of a method for producing washable absorbent garments,
- Figure 12 is a schematic perspective view showing an embodiment of the welding unit indicated by the arrow XII in figure 5,
- Figure 13 is a perspective view of the part indicated by the arrow XIII in figure 12,
- Figure 14 is a perspective view of an anvil indicated by the arrow XIV in figure 13,
- Figure 15 is a cross-section taken along the line XV-XV of figure 14,
- Figure 16 is a cross-section shoving an alternative embodiment of the anvil of figure 15,
- Figure 17 is a schematic view showing another embodiment of the welding unit indicated by the arrow XII in figure 5,
- Figure 18 is a schematic cross-section of a laser beam indicated by the arrow XVIII in figure 17, and
- Figure 19 is a diagram showing energy shape distribution vs. radius in the laser beam of figure 18.

It will be appreciated that the various figures may not be represented on the same scale. It will also be appreciated that in some figures certain elements or components may not be shown for a better understanding.

### Detailed description

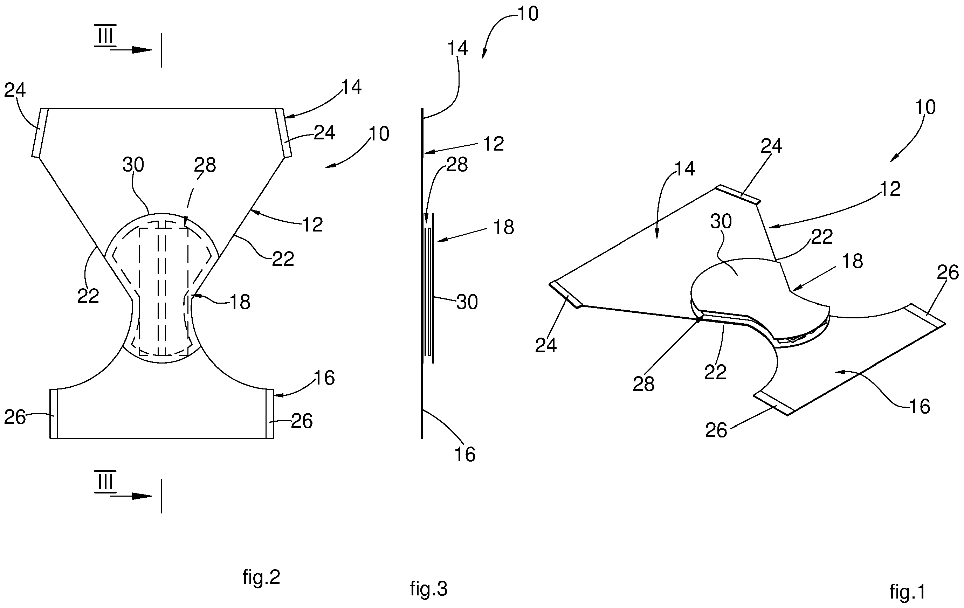

With reference to figures 1-3 the numeral reference 10 indicates a washable absorbent garment.

The washable absorbent garment 10 comprises a back layer 12 of washable fabric forming a pant structure. The back layer 12 has rear and front waist sections 14, 16 and a crotch section 18 intermediate between the rear and front waist sections 14, 16. The rear and front waist sections 14, 16 are wider than the crotch section 18, such that in the extended position of figures 1 and 2 the back layer 12 has substantially an hourglass shape.

The back layer 12 is a fabric formed by woven threads which is washable as ordinary textile garments, e.g. swimsuits, underwear, etc. The back layer 12 may be made of any natural or synthetic material used for producing textile articles, e.g. cotton, Lycra, nylon, polyester, polyamide, elastane, etc. and any mixture thereof. In possible embodiments, the back layer 12 may be an elastic fabric made of a mixture of cotton and elastane, e.g. 80% cotton and 20% elastane or 80% polyamide and 20% elastane. The back layer 12 may have a weight comprised between 100 - 250 g/m², preferably between 150-200 g/m² and preferably between 160-190 g/m².

The crotch section 18 of the back layer 12 has two curved side edges 22 shaped to conform to the legs of the user in the configuration in which the washable absorbent garment 10 is worn.

The rear and front waist sections 14, 16 of the back layer 12 have respective side edges 24, 26 which are joined to each other such that the washable absorbent garment 10 is closed in a pant-like shape and has two leg openings on opposite sides of the crotch section 18. The side edges 24, 26 are joined to each other by ultrasonic welding, laser welding or glue as it will be disclosed in detail in the following.

The washable absorbent garment 10 may comprise a washable absorbent core 28 permanently fixed in the crotch section 18 of the back layer 12. The washable absorbent core 28 extends only on the crotch section 18 of the back layer 12 and does not extend on the rear and front waist sections 16, 18 of the back layer 12.

The washable absorbent core 28 includes a washable absorbent pad comprising washable superabsorbent fibers. A material suitable for producing the washable absorbent pad 30 may be that marketed by Technical Absorbents Ltd (UK) under the trade name SAF^{®}. The washable absorbent pad may be formed by needlefelt, thermal bonded, laminated non-woven fibers, formed by polyester, polypropylene and SAF^{®} (Sodium polyacrylate - crosslinked polymers or copolymers of carboxylic acid salts - in fibers).

The washable absorbent core 28 may include an acquisition and diffusion layer (ADL) and a barrier film. The washable absorbent pad may be sandwiched between the acquisition and diffusion layer and the barrier film.

The washable absorbent garment 10 may comprise a top layer 30 of washable fabric which covers the washable absorbent core 28. The top layer 30 may be made of the same material as the fabric forming the back layer 12. The top layer 30 completely covers the washable absorbent core 28 but does not extend on the rear and front waist sections 16, 18 of the back layer 12.

The top layer 30 and the washable absorbent core 28 may be permanently joined to the back layer 12 by welding, e.g. ultrasonic welding, or glue.

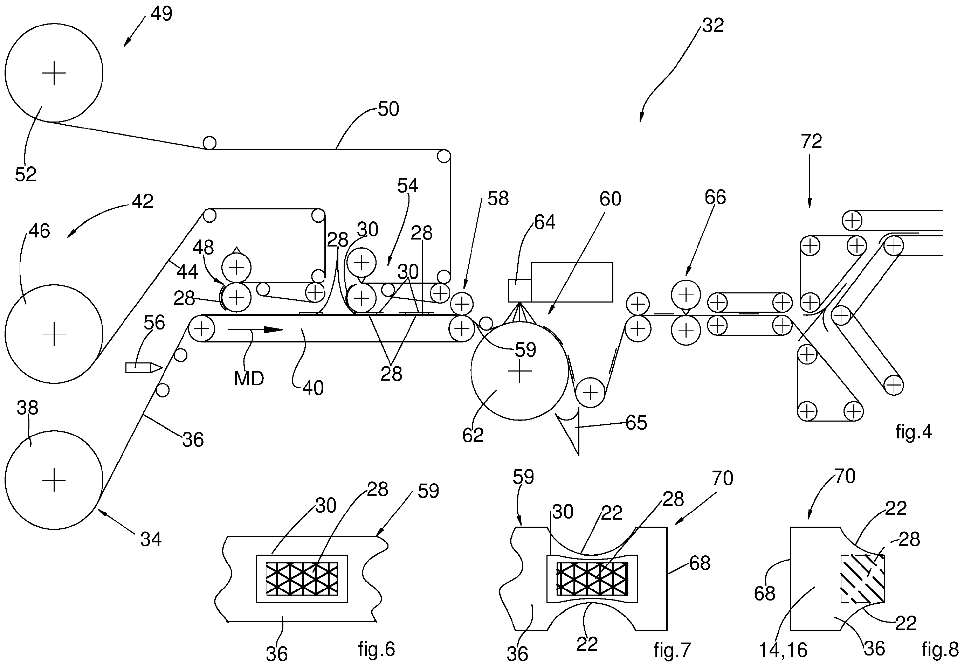

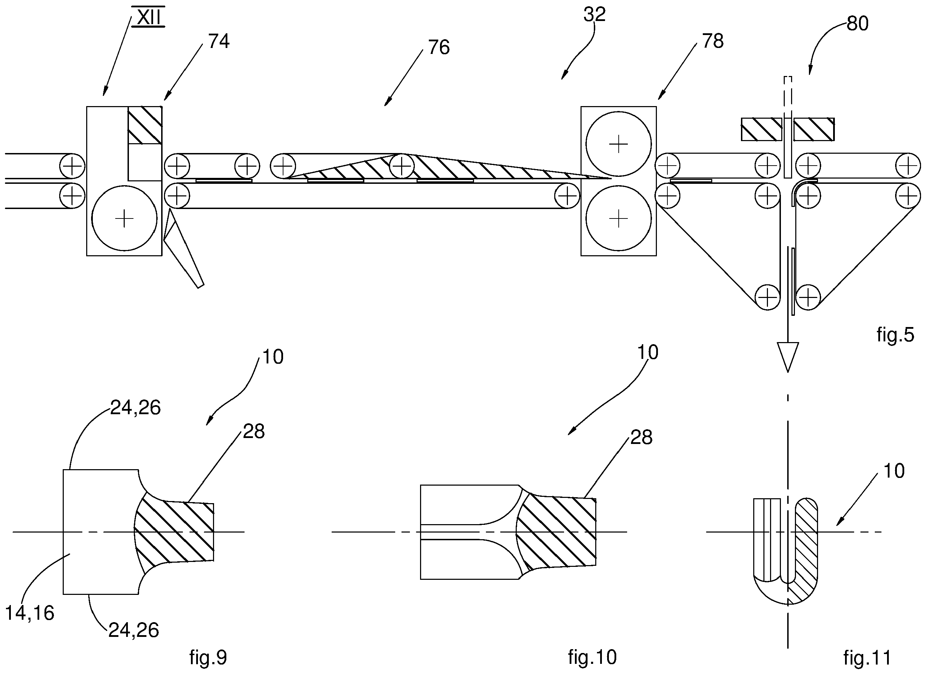

An embodiment of an apparatus for producing the washable absorbent garment 10 of the type illustrated in Figures 1-3 is shown in figures 4 and 5 and is indicated by the reference numeral 32.

With reference to figure 4, the apparatus 32 comprises a first unwinding unit 34 configured for unwinding a first continuous fabric layer 36 from a first reel 38. The first continuous fabric layer 36 is advanced in a machine direction MD on a conveyor 40.

The apparatus 32 may comprise a second unwinding unit 42 configured for unwinding a continuous absorbent tape 44 from a second reel 46. The apparatus 32 may comprise a first cut-and-slip unit 48 configured for transversally cutting the continuous absorbent tape 44 to form individual absorbent cores 28. The first cut-and-slip unit 48 is configured for longitudinally spacing from each other the individual absorbent cores 28 and for applying the individual absorbent cores 28 on the first continuous fabric layer 36 in longitudinally spaced positions on the conveyor 40.

The apparatus 32 may comprise a third unwinding unit 49 configured for unwinding a second continuous fabric layer 50 from a third reel 52. The apparatus 32 may comprise a second cut-and-slip unit 54 configured for transversally cutting the second continuous fabric layer 50 to form individual top layers 30. The second cut-and-slip unit 54 is configured for longitudinally spacing from each other the individual top layers 30 and for applying the individual top layers 30 on respective absorbent cores 28 previously applied on the first continuous fabric layer 36 on the conveyor 40.

The apparatus 32 may comprise a glue dispenser 56 configured for applying a discrete pattern of glue on the first continuous fabric layer 36 for attaching the individual absorbent cores 28 and the individual top layers 30 on the first continuous fabric layer 36.

The apparatus 32 may comprise a sealing press 58 located at the end of the conveyor 40, configured for compressing the individual absorbent cores 28 and the individual top layers 30 to the first continuous fabric layer 36.

After the compression, the individual absorbent cores 28 and the individual top layers 30 are permanently fixed by glue to the first continuous fabric layer 36 in longitudinally spaced positions to form a continuous garment tape 59. Alternatively, the apparatus 32 may comprise an ultrasonic welding unit configured for permanently attaching by ultrasonic welding the individual absorbent cores 28 and the individual top layers 30 on the first continuous fabric layer 36.

Figure 6 shows a portion of the continuous garment tape 59 formed by individual absorbent cores 28 and individual top layers 30 fixed to the first continuous fabric layer 36.

The apparatus 32 comprises a cutting unit 60 configured for cutting longitudinal side edges of the continuous garment tape 59 as it advances in the machine direction MD along curved profiles shaped to conform to the legs of the user. The cutting unit 60 comprises an anvil roller 62 and a cutting tool 64 cooperating with the outer surface of the anvil roller 62. The cutting tool 64 may be a laser nozzle or an ultrasonic horn configured for carrying out a laser or ultrasonic cut of the first continuous fabric layer 36. The cutting tool 64 may also cut simultaneously side edges of the top layer 30 and of the absorbent core 28.

The cutting tool 64 may be movable transversally to the machine direction to form, in conjunction with the longitudinal movement of the continuous garment tape 59, curved cuts which form curved side edges 22 (figure 7) of the continuous garment tape 59. The portions trimmed from the continuous garment tape 59 are collected in a waste collecting channel 65.

The apparatus 32 comprises a transverse cutting unit 66 configured to cut the first continuous fabric layer 36 along transverse cutting lines 68 (figure 7) to form individual blank absorbent garments 70. Figure 7 shows the continuous garment tape 59 after the longitudinal and transverse cutting steps.

The apparatus 32 comprises a transverse folding unit 72 configured for folding the individual blank absorbent garments 70 along respective transverse folding lines, so as to overlap to each other the rear and front waist sections 14, 16 of each blank absorbent garment 70. Figure 8 shows a blank absorbent garment 70 after the transverse folding step.

With reference to figure 5, the apparatus 32 comprises a welding unit 74 which receives the folded blank absorbent garments 70 from the folding unit 72. With reference to figure 9, the welding unit 74 is configured for sealing side edges 24, 26 of the rear and front waist sections 14, 16 of the blank absorbent garments 70, to form finished absorbent garments 10.

The apparatus 32 may comprise a linear folding unit 76 configured for folding side portions of the finished absorbent garments 10 as shown in figure 10.

The apparatus 32 may comprise a final press unit 78 configured for pressing the folded portions of the finished absorbent garments 10.

The apparatus 32 may comprise a final folding unit 80 configured for carrying out a final folding of the finished absorbent garments 10 as shown in figure 11. After the final folding unit 80 the finished absorbent garments 10 may be sent to a packaging machine.

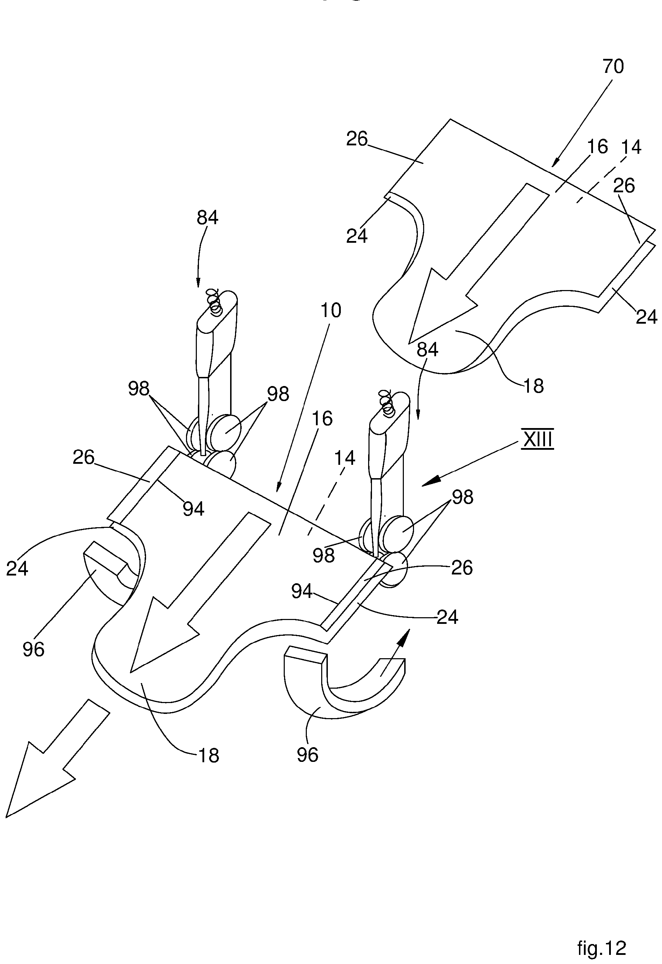

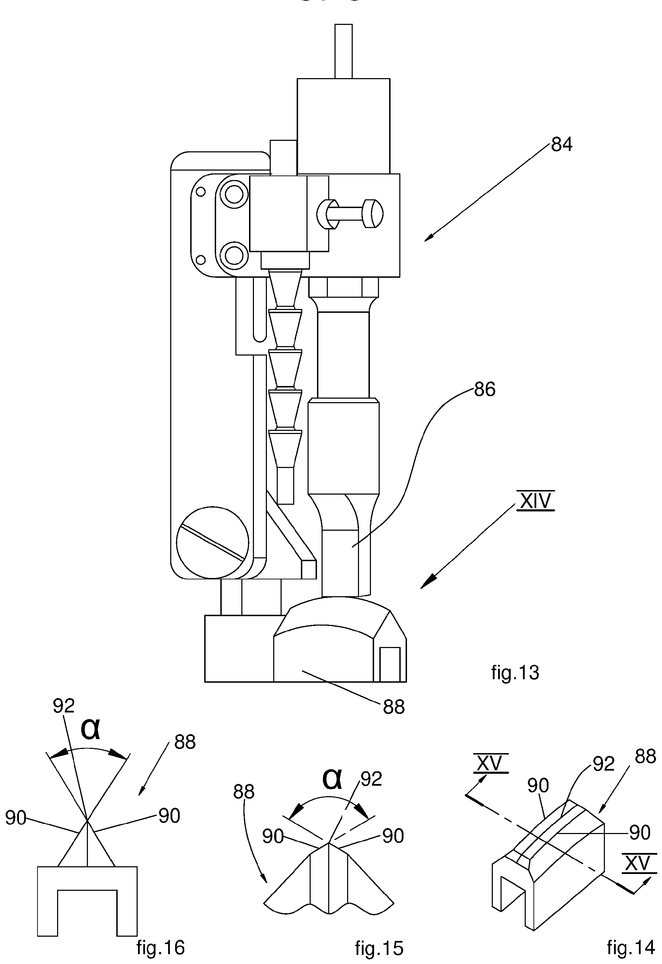

Figures 12 and 13 show an embodiment of the welding unit 74 which carries out ultrasonic welding of the overlapped side edges 24, 26 of the blank absorbent garments 70.

The welding unit 74 comprised a pair of ultrasonic welding devices 84, each comprising an ultrasonic horn 86 and an anvil 88. The anvil 88 may be a stationary element or a rotating anvil roller.

With reference to figures 14, 15, 16, the anvil 88 has two inclined welding surfaces 90 converging on a sharp edge 92. The sharp edge 92 may be parallel or slightly inclined to the moving direction X (figure 12) of the blank absorbent garments 70. The angle α between the two inclined welding surfaces 90 may be comprised between 90° - 160°.

The blank absorbent garments 70 advance in the moving direction X and each of the two opposite overlapped edges 24, 26 passes between the ultrasonic horn 86 and the anvil 88 of a respective ultrasonic welding device 84. The overlapped side edges 24, 26 are welded along respective welding lines 94 while advancing the blank absorbent garments 70 in the moving direction X. The overlapped side edges 24, 26 are simultaneously cut on the respective sharp edges 92 of the respective anvils 88 flush with the respective welding lines 94. The portions of the side edges 24, 26 external to the welding lines 94 are disposed as scrap. Each ultrasonic welding device may comprise a suction channel 96 for aspirating the scraps.

In possible embodiments, to obtain an inclined cutting/sealing line on final products, the ultrasonic welding devices 84 may be moved simultaneously in cross-direction.

After the welding and the simultaneous cut of the side edges 24, 26, finished washable absorbent garments 10 are obtained. The ultrasonic welding and simultaneous cut of the side edges 24, 26 provides washable absorbent garments 10 with a very smooth surface and without an increased thickness at the sides. The ultrasonic welding and simultaneous cut of the side edges 24, 26 also provides great flexibility when the shape and dimensions of the washable absorbent garments 10 changes.

With reference to figure 12, to improve the sealing quality, each ultrasonic welding device 84 may comprise two pairs of nipping rollers 98, configured for compressing the overlapped side edges 24, 26 on opposite sides of the respective welding line 94.

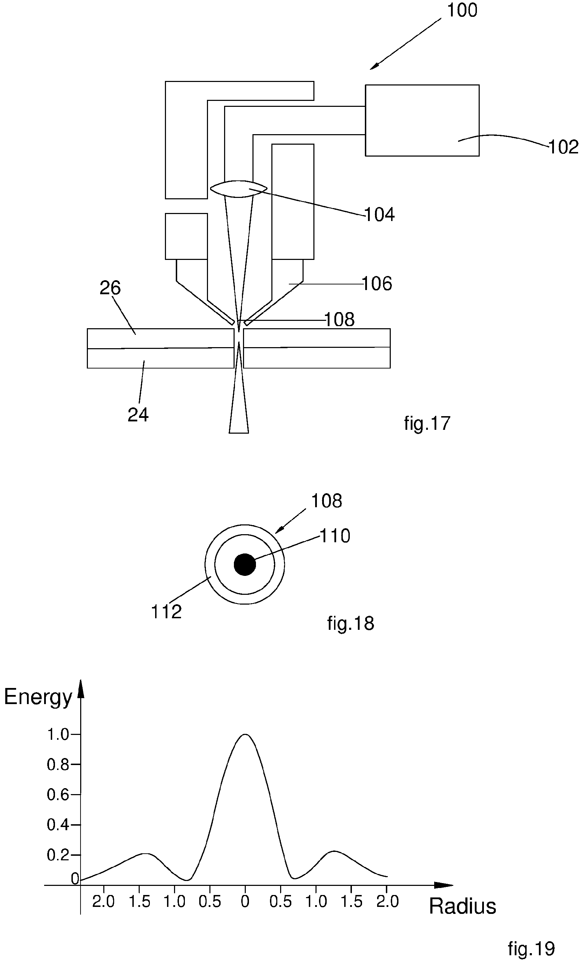

Figure 17 shows an embodiment of the welding unit 74 which carries out laser welding of the overlapped side edges 24, 26 of the blank absorbent garments 70. The welding unit 74 comprises a pair of laser welding devices 100 each of which comprises a laser resonator 102, a focusing lens 104 and a nozzle 106. With reference to figures 17 and 18, the focusing lens 104 is configured for generating a laser beam 108 having a central area 110 with a first energy density and an annular area 112 with a second energy density and surrounding the central area 110.

The graph of figure 19 shows the energy density distribution (i.e. energy per surface unit) vs. the radius of the laser beam 108. The first energy density of the central area 110 is configured for carrying out the cut of the two overlapped side edges 24, 26. The second energy density of the annular area 112 is configured for carrying out laser welding of the two overlapped side edges 24, 26.

In operation, the apparatus 32 implements a method for producing washable absorbent garments 10, comprising:
- providing blank washable absorbent garments 70 each comprising a fabric back layer 12 having rear and front waist sections 14, 16 and a crotch section 18 intermediate between the rear and front waist sections 14, 16, wherein the rear and front waist sections 14, 16 have respective said side edges 24, 26,
- folding said blank washable absorbent garments 70 and overlapping to each other said side edges 24, 26 of said rear and front waist sections 14, 16,
- advancing in a moving direction X said folded blank washable absorbent garments 70, and
- welding said overlapped side edges 24, 26 along respective welding lines 94 while advancing said folded blank washable absorbent garments 70 in said moving direction X and simultaneously cutting said side edges 24, 26 flush with the respective welding lines 94.

The method and apparatus according to the present invention may have a production rate up to 200 pieces/1', which is a huge progress as compared to the production rate of prior art washable absorbent products which is about 1 piece/1'.

Of course, without prejudice to the principle of the invention, the details of construction and the embodiments can be widely varied with respect to those described and illustrated, without thereby departing from the scope of the invention as defined by the claims that follow.

## Claims

1. A method for producing washable absorbent garments (10), comprising:
- providing blank washable absorbent garments (70) each comprising a fabric back layer (12) having rear and front waist sections (14, 16) and a crotch section (18) intermediate between the rear and front waist sections (14, 16), wherein the rear and front waist sections (14, 16) have respective said side edges (24, 26),
- folding said blank washable absorbent garments (70) and overlapping to each other said side edges (24, 26) of said rear and front waist sections (14, 16),
- advancing in a moving direction (X) said folded blank washable absorbent garments (70), and
- welding said overlapped side edges (24, 26) along respective welding lines (94) while advancing said folded blank washable absorbent garments (70) and simultaneously cutting said side edges (24, 26) flush with the respective welding lines (94).

2. The method of claim 1, comprising disposing as scrap portions of said side edges (24, 26) external to said welding lines (94).

3. The method of claim 1 or claim 2, wherein said overlapped side edges (24, 26) are ultrasonic welded on an inclined welding surface (90) converging on a sharp edge (92) and are ultrasonic cut on said sharp edge (92).

4. The method of claim 3, wherein said overlapped side edges (24, 26) are passed between an ultrasonic horn (86) and an anvil (88), the anvil (88) having two inclined welding surfaces (90) converging on said sharp edge (92).

5. The method of claim 4, wherein the angle between said inclined welding surfaces (90) is comprised between 90-160°.

6. The method of any of the preceding claims, comprising compressing portions of said overlapped side edges (24, 26) opposite to respective welding lines (94) while welding and simultaneously cutting said side edges (24, 26).

7. The method of claim 6, wherein each of said overlapped side edges (24, 26) is compressed between two pairs of nipping rollers (98).

8. The method of claim 1 or claim 2, wherein said overlapped side edges (24, 26) are laser welded and simultaneously laser cut.

9. The method of claim 6, wherein said overlapped side edges (24, 26) are subjected to a laser beam (108) having a central area (110) configured for cutting said overlapped side edges (24, 26) and an annular area (112) surrounding said central area (110) and configured for welding said overlapped side edges (24, 26).

10. The method of any of the preceding claims, wherein said fabric back layer (12) has a weight comprised between 100-250 g/m², preferably between 150-200 g/m² and preferably between 160-190 g/m².

11. The method of any of the preceding claims, comprising:
- providing a first continuous fabric layer (36) having two opposite linear side edges,
- advancing said first continuous fabric layer (36) in a longitudinal direction,
- cutting said opposite side edges of said first continuous fabric layer (36) along shaped profiles while advancing said first continuous fabric layer (36) in said longitudinal direction,
- transversely cutting said first continuous fabric layer (36) to form individual blank pant-like washable absorbent garments (70).

12. A washable absorbent garment (10) comprising a back layer (12) of washable fabric forming a pant-like structure and having rear and front waist sections (14, 16) and a crotch section (18) intermediate between the rear and front waist sections (14, 16), wherein the rear and front waist sections (14, 16) have respective side edges (24, 26) joined to each other by welding along welding lines (94) and wherein said side edges (24, 26) are cut flush with said welding lines (94).

13. An apparatus for producing washable absorbent garments (10), comprising:
- a first unwinding unit (34) configured for unwinding a first continuous fabric layer (36) from a first reel (38) and for advancing said first continuous fabric layer (36) in a longitudinal direction,
- a side cutting unit (60) configured for cutting longitudinal side edges of said first continuous fabric layer (36) along shaped profiles,
- a transverse cutting unit (66) configured for cutting said first continuous fabric layer (36) along transverse cutting lines (68) to form individual blank pant-like garments (70),
- a transverse folding unit (72) configured for folding the individual blank pant-like garments (70) along respective transverse folding lines, and
- a welding unit (74) configured for welding overlapped side edges (24, 26) of said folded blank pant-like garments (70) along respective welding lines (94) while advancing said folded blank washable absorbent garments (70) in a moving direction (X) and for simultaneously cutting said side edges (24, 26) flush with the respective welding lines (94).

14. The apparatus of claim 13, wherein the welding unit (74) comprises a pair of ultrasonic welding devices (84) each including an ultrasonic horn (86) cooperating with a respective anvil (88), wherein each anvil (88) has two inclined welding surfaces (90) converging on a sharp edge (92).

15. The apparatus of claim 13, wherein the welding unit (74) comprises a pair of laser welding devices (100) configured for generating a laser beam (108) having a central area (110) configured for cutting said overlapped side edges (24, 26) and an annular area (112) surrounding said central area (110) configured for welding said overlapped side edges (24, 26).
